# EUROPEAN PATENT SPECIFICATION

(11) **EP 2 182 954 B1**
(45) Date of publication and mention of the grant of the patent: **28.11.2018**
(21) Application number: 08775321.6
(22) Date of filing: 24.07.2008
(51) Int. Cl.: A61K 31/56, A61P 9/10, A61K 9/00

(54) **USE OF NOR-BILE ACIDS IN THE TREATMENT OF ARTERIOSCLEROSIS**
VERWENDUNG VON NOR-GALLENSÄUREN BEI DER BEHANDLUNG VON ARTERIOSKLEROSE
UTILISATION D'ACIDES NOR-BILIAIRES DANS LE TRAITEMENT DE L'ARTÉRIOSCLÉROSE

(30) Priority: 25.07.2007 US 951728 P; 25.07.2007 EP 07113107
(43) Date of publication of application: 12.05.2010
(73) Proprietor: Medizinische Universität Graz, 8036 Graz (AT)
(72) Inventor: TAREK, Moustafa, A-8010 Graz (AT); TRAUNER, Michael, A-8010 Graz (AT); FICKERT, Peter, A-8010 Graz (AT)
(74) Representative: Bühler, Dirk
(86) International application number: PCT/EP2008/059704
(87) International publication number: WO 2009/013334

(56) References cited:
- WO-A-94/00155
- WO-A-99/52932
- WO-A-02/083147
- WO-A-2006/119803
- WO-A-2007/061820
- US-A- 5 512 558
- FICKERT P ET AL: "98 DIFFERENTIAL EFFECTS OF NORUDCA AND UDCA IN THE TREATMENT OF FATTY LIVER AND ARTERIOSCLEROSIS IN WESTERN CHOW-FED APOE KNOCK OUT MICE" JOURNAL OF HEPATOLOGY, MUNKSGAARD INTERNATIONAL PUBLISHERS, COPENHAGEN, DK, vol. 48, April 2008 (2008-04), page S42, XP022626254 ISSN: 0168-8278 [retrieved on 2008-04]
- FICKERT PETER ET AL: "Primary sclerosing cholangitis - The arteriosclerosis of the bile duct?" LIPIDS IN HEALTH AND DISEASE, BIOMED CENTRAL, LONDON, GB, vol. 6, no. 1, 25 January 2007 (2007-01-25), page 3, XP021024437 ISSN: 1476-511X

## Description

### FIELD OF THE INVENTION

The present invention relates to the field of treatment and/or prevention of arteriosclerosis. In particular, the invention relates to nor-ursodeoxycholic acid for use in treating and/or preventing arteriosclerosis.

### BACKGROUND OF THE INVENTION

Atherosclerosis is a progressive disease characterized by the accumulation of lipids, fibrous elements and cells in the large arteries, which can ultimately lead to the formation of atherosclerotic plaques.

Plaque formation in atherosclerosis has twofold implications. First, athermatous plaques may eventually rupture and lead to stenosis of the artery and therefore an insufficient blood supply in the organ in need thereof. Consequences can be e.g. an infarction of various organs, most importantly of the heart (mycocardial infarction) and brain (stroke). Second, the artery enlargement process, which is a response to plaque formation, may become excessive and result in an aneurysm.

Atherosclerosis is known to be one case of a disease type commonly denoted as arteriosclerosis, i.e. the hardening of arteries resulting from depositions of lipids, collagen and other fibrous elements. Another sub-type of the general condition arteriosclerosis is the disease arteriolosclerosis in which a hardening of the small arteries is observed as a result of *inter alia* collagen deposition, muscle wall thickening and deposition of proteins.

Arteriosclerosis and particularly atherosclerosis are one of the leading causes of death at least in the western hemisphere. In Western Europe and the United States it is recognised as the most common and deadliest disease besides cancer. The disease mechanisms underlying arteriosclerosis and particularly atherosclerosis have been at the focus of intense research over the last decades and are reviewed constantly in various publications. Representative overviews on current understanding of the molecular mechanisms underlying and contributing to atherosclerosis can e.g. be found in Libby et. al. (Nature, 2002, 420, 868-874), Lusis et.al. (Nature, 2000, 407, 233-241) and Ross et. al. (New England Journal of Medicine, 1999, 340(2) 115-126).

Treatment and/or prevention of atherosclerosis as being the most prominent example of arteriosclerosis includes avoidance of environmental risk factors and medical treatments.

Typical risk factors for atherosclerosis include high levels of cholesterol in the blood, high blood pressure, diabetes, obesity, and physical inactivity.

Furthermore, it has been suggested recently (see e.g. Targher G. et Arcaro G. in Atherosclerosis, 2007, 191, 235-240) that non-alcoholic fatty liver disease (NAFLD) is likely to be associated with increased cardiovascular disease (CVD) risk raising the possibility that NAFLD may be an early mediator of atherosclerosis. NAFLD is present in up to one-third of the general population and in the majority of patients with cardio-metabolic risk factors. It seems that the severity of liver histology in NAFLD patients is closely associated with markers of early atherosclerosis.

Thus, it seems that NAFLD represents another risk factor for the development of atherosclerosis.

These risk factors can be addressed by a different lifestyle including a changed diet, increased sports activity and reduced smoking.

The second line of treatment for atherosclerosis includes use of medicinal products with treatment by statins being the prominent one. Statins mainly aim at reducing high cholesterol blood levels and thus have a rather prophylactic effect. Most of the statins currently available are not suitable, for example, to reduce atherosclerotic lesions and/or plaques.

Further, some of the medications that are currently used for treatment of atherosclerosis have considerable side effects.

WO 2007/061820 entitled "Dissolution of arterial cholesterol plaques by a class of pharmacological compounds" is inter alia concerned with a pharmacological substance, namely a biliary salt or acid or precursor or derivative with emulsifying properties administered into the systemic circulation of a patient via a variety of routes of administration including oral administration.

There is thus a continuing need for drugs that can be used in the treatment and/or prevention of arteriosclerosis and particularly of atherosclerosis.

Furthermore, with regard to the correlation of risk factors (particularly NAFLD) and atherosclerosis, there is a need for drugs that can be used in the treatment and/or prevention of arteriosclerosis and particularly atherosclerosis and at the same time in the treatment and/or prevention of said risk factors (particularly NAFLD).

### SUMMARY OF THE INVENTION

It is the objective of the present invention to provide for nor-ursodeoxycholic acid and/or at least one pharmaceutically acceptable salt thereof for use in the treatment of arteriosclerosis and particularly atherosclerosis as claimed in independent claim 1.

According to the disclosure, one aspect relates to at least one nor-bile acid and/or at least one pharmaceutically acceptable salt, ester and/or derivative thereof for the treatment and/or prevention of arteriosclerosis.

According to the disclosure, one aspect relates to pharmaceutical compositions comprising at least one nor-bile acid and/or at least one pharmaceutically acceptable salt, ester and/or derivative thereof for the treatment and/or prevention of arteriosclerosis.

According to the disclosure, one aspect relates to the use of at least one nor-bile acid and/or at least one pharmaceutically salt, ester and/or derivative thereof in the manufacture of a medicament for the treatment and/or prevention of arteriosclerosis.

According to the disclosure, one aspect relates to a method of treating and/or preventing arteriosclerosis in a human or animal subject comprising the step of administering at least one nor-bile acid and/or at least one pharmaceutically acceptable salt, ester and/or derivative thereof. In one embodiment, nor-bile acids may have the following formula (I): 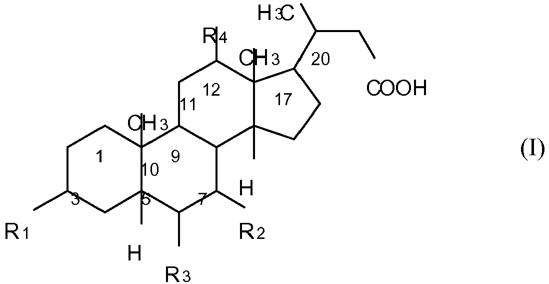
with R₁ being -OH or -H,
with R₂ being -OH or -H,
with R₃ being - OH or -H, and
with R₄ being - OH or -H,
wherein the OH-groups of R₁, R₂, R₃ or R₄ are in α or β conformation.

According to the disclosure in one aspect, the nor-bile acid is selected from the group consisting of:
24-nor[3α, 7α-dihydroxy-5β-cholan-23-oic] acid (being also designated as nor-chenodeoxycholic acid), 24-nor[3α, 7β-dihydroxy-5β-cholan-23-oic] acid (being also designated as nor-ursodeoxycholic acid (nor-UDCA), 24-nor[3α, 12α-dihydroxy-5β-cholan-23-oic] acid (being also designated as nor-deoxycholic acid, 24-nor[3α-hydroxy-5β-cholan-23-oic] acid (being also designated as nor-lithocholic acid), 24-nor[3α, 7α, 12α-trihydroxy-5β-cholan-23-oic] acid (being also designated as nor-cholic acid), 24-nor[3α, 12β-dihydroxy-5β-cholan-23-oic] acid and 24-nor[3α, 6β-dihydroxy-5β-cholan-23-oic] acid.

The numbering is based on the numbers provided for formula (I).

According to the invention the at least one nor-bile acid is nor-ursodeoxycholic acid, i.e. nor-UDCA. One will consider to use about 10 to about 8,000 mg, about 25 to about 5,000 mg, about 50 to about 1,500 mg or about 250 to about 500 mg of at least nor-UDCA or at least one pharmaceutically acceptable salt and/or ester thereof for the pharmaceutical compositions, for use in treating and/or preventing arteriosclerosis as mentioned above.

The treatment and/or prevention of atherosclerosis constitutes a preferred embodiment of the disease picture summarized by the term arteriosclerosis in the context of the present invention.

A particularly preferred embodiment of the present invention relates to pharmaceutical compositions comprising nor-UDCA and/or at least one pharmaceutically acceptable salt and/or ester thereof for the treatment and/or prevention of atherosclerosis. Another particularly preferred embodiment relates to the use of nor-UDCA and at least one pharmaceutically acceptable salt and/or ester thereof in the manufacture of a medicament for the treatment and/or prevention of atherosclerosis.

Nor-UDCA and/or at least one pharmaceutically acceptable salt and/or ester thereof may be applied in the amounts mentioned above.

According to the invention, nor-UDCA is formulated in an oral dosage form. One will consider to use about 10 to about 8,000 mg, about 25 to about 5,000 mg, about 50 to about 1,500 mg or about 250 to about 500 mg of nor-UDCA or at least one pharmaceutically acceptable salt and/or ester thereof for the pharmaceutical compositions for use in treating and/or preventing arteriosclerosis as mentioned above.

### BRIEF DESCRIPTION OF THE FIGURES

Figure 1: experimental setup for data of treatment studies described in Experiment 1 and as depicted in figures 2 and 3
Figure 2: Nor-UDCA reduces atherosclerosis in ApoE- /- mice in a treatment study
   A): Histological analysis of red oil-stained aorta sections of ApoE-/- mice raised on a western chow diet (21% w/w fat, 1.5% w/w cholesterol). B): Histological analysis of red oil-stained aorta sections of ApoE-/-mice fed on a western chow diet containing 0.5% w/w nor-UDCA. Arrows indicate regions of reduced plaque size.

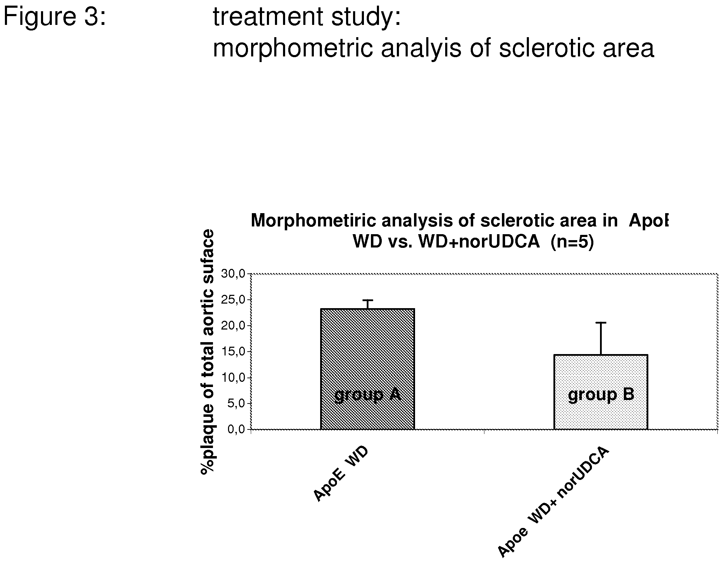

Figure 3: Nor-UDCA reduces atherosclerosis in ApoE- /- mice in a treatment study Morphometric analysis of sclerotic area in ApoE - /- mice which were fed with a western chow diet for 8 weeks plus 4 weeks either without nor-UDCA (12 weeks in total) (left bar) or with 0.5% w/w nor-UDCA (right bar).

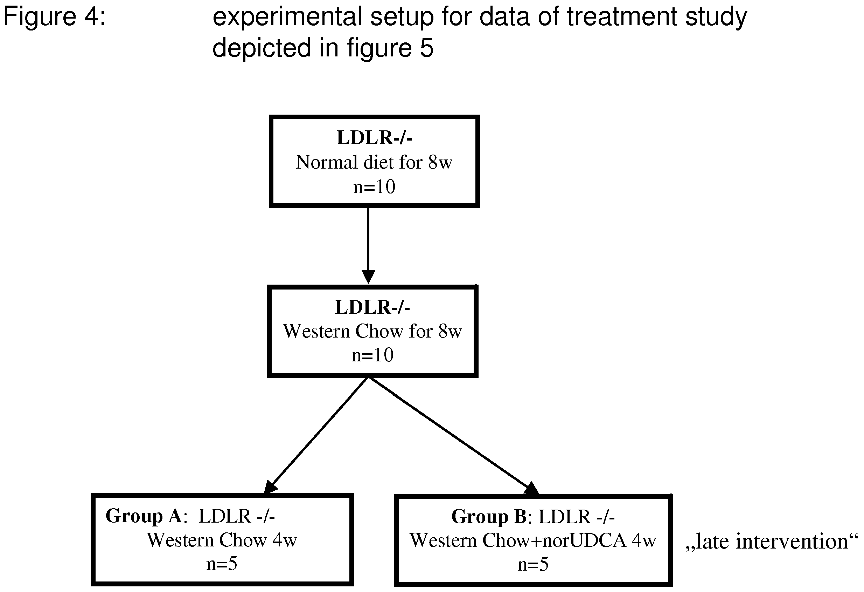

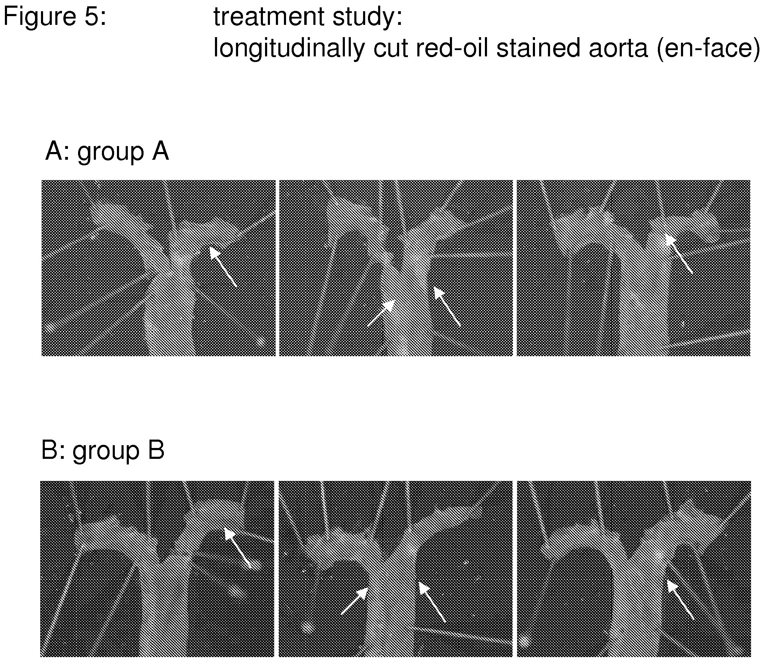

Figure 4: experimental setup for data of treatment study described in Example 2 and as depicted in figure 5
Figure 5: Nor-UDCA reduces atherosclerosis in LDLR-/-mice in a treatment study
   A): histological analysis (en face) on arteries of LDLR-/-mice. The aorta of LDLR-/-mice was cut longitudinally and subsequently en-face stained with red oil. The pictures show the en-face red oil stained aorta of LDLR-/-mice fed with a western chow diet. bB): shows aortas from LDLR-/mice fed with a western chow diet and 0.5% w/w nor-UDCA (magnification for the upper and lower panel are 40-fold). Arrows indicate regions of reduced plaque size.

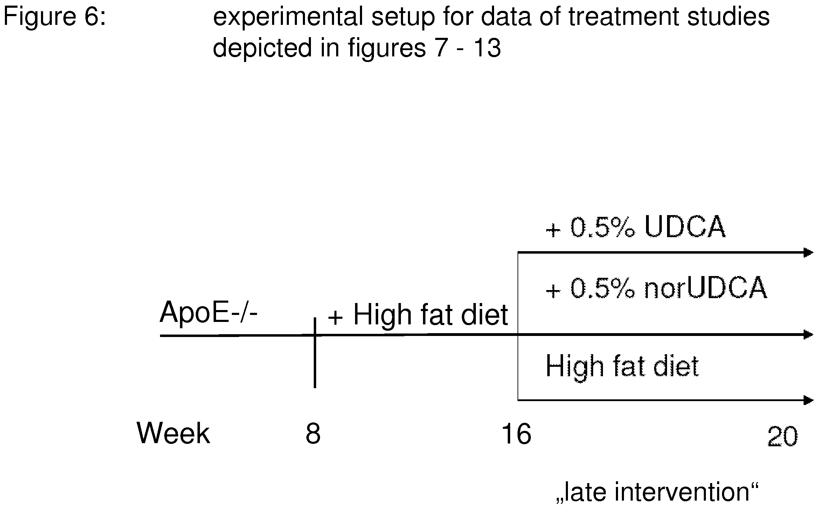

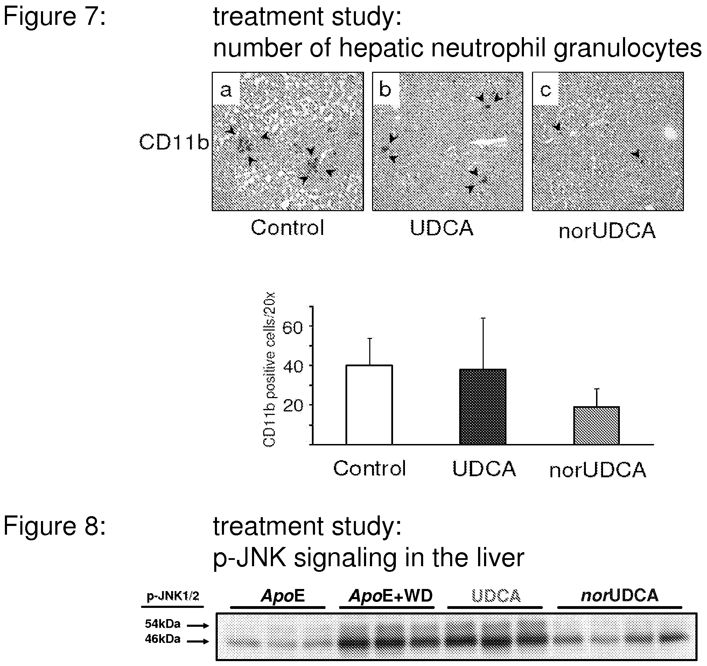

Figure 6: experimental setup for data of treatment studies described in Experiment 3 and as depicted in figures 7 to 13
Figure 7: Nor-UDCA reduces the number of hepatic neutropohil granulocytes in a treatment study
   Top: immunohistochemical analysis for the number of CD11b-positive cells (corresponding to neutropohil granulocytes) in liver preparations; Bottom: quantitative analysis of CD11b positive cells in the liver by counting of said cells in 5 mice of each group.
Figure 8: Nor-UDCA reduces the p-JNK levels in the liver in a treatment study
   Western blot analysis of different liver homogenates as indicated from either normally fed ApoE-/- mice, ApoE-/- mice fed with western diet, ApoE-/- mice fed with western diet and UDCA or ApoE-/- mice fed with western diet and nor-UDCA (see also figure 6) for p-JNK.

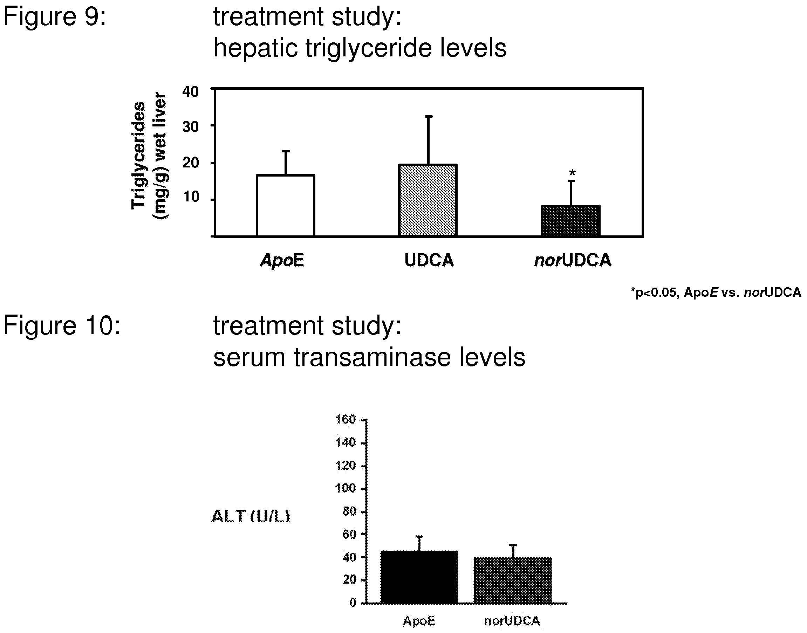

Figure 9: Nor-UDCA reduces hepatic triglyceride levels in a treatment study
   The hepatic triglyceride levels were determined for 5 mice of each group as outlined in figure 6.
Figure 10: Nor-UDCA slightly reduces ALT-levels in a treatment study
   The serum alanin-amino-transferase levels were determined for 5 mice of the group fed with western diet only and for 5 mice of the group fed with western diet comprising additionally nor-UDCA.

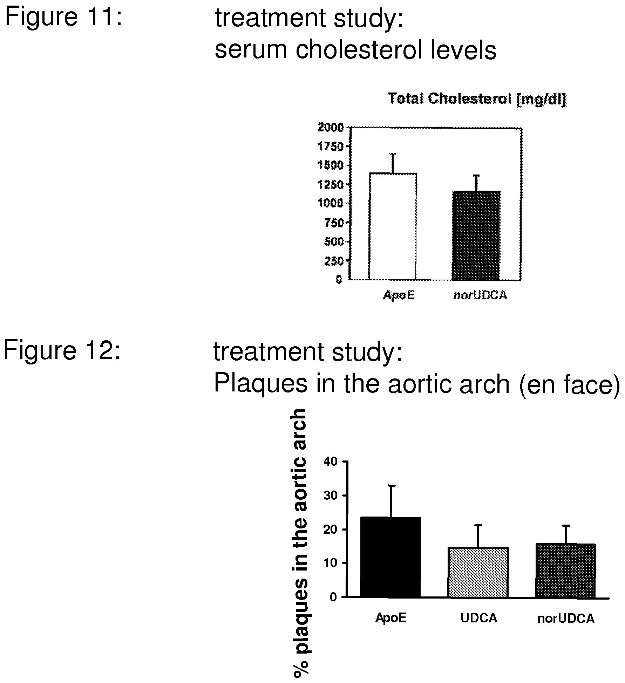

Figure 11: Nor-UDCA slightly reduces serum cholesterol levels in a treatment study
   The serum cholesterol levels were determined for 5 mice of the group fed with western diet only and for 5 mice of the group fed with western diet comprising additionally nor-UDCA.
Figure 12: Nor-UDCA reduces plaques in the aortic arch in a treatment study
   Longitudinal sections of the aorta were en-face stained with red oil and the area comprising plaques was determined and expressed as % plaques of the total area. The analysis was done for 5 mice in each group as depicted.

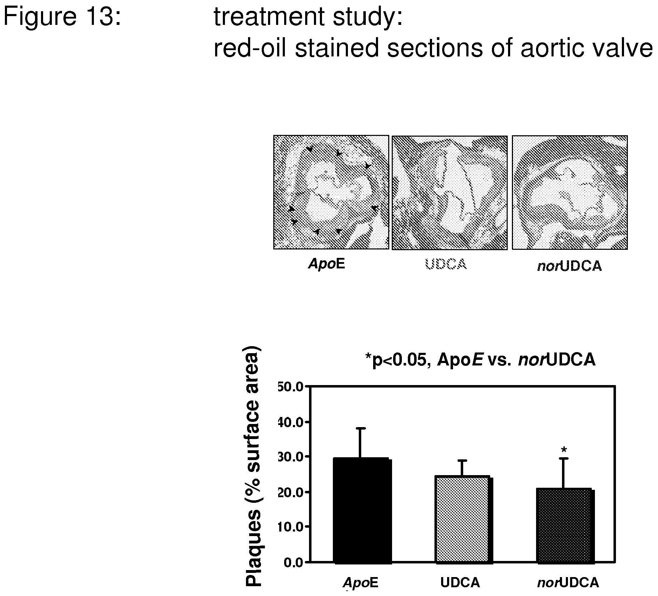

Figure 13: Nor-UDCA reduces plaques in the aortic valve in a treatment study Cross-sections of the aortic valve were stained with red-oil and the area comprising plaques was determined and expressed as % plaques of the total surface area. The analysis was done for 5 mice in each group as depicted.

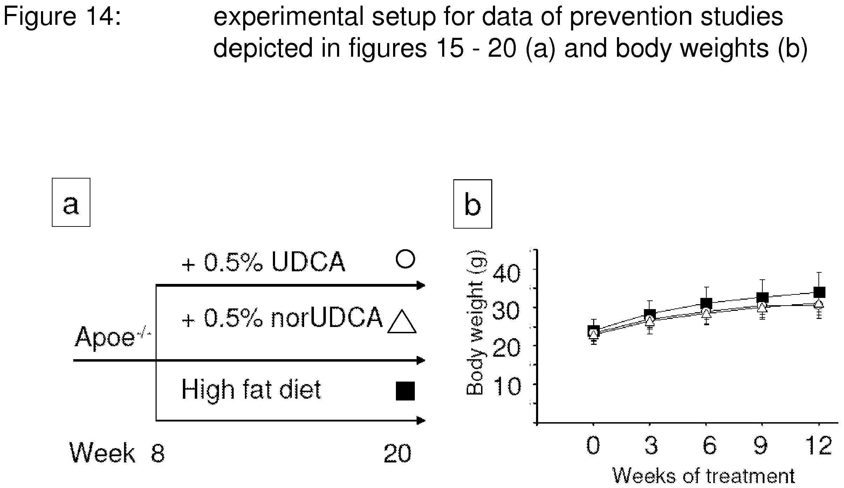

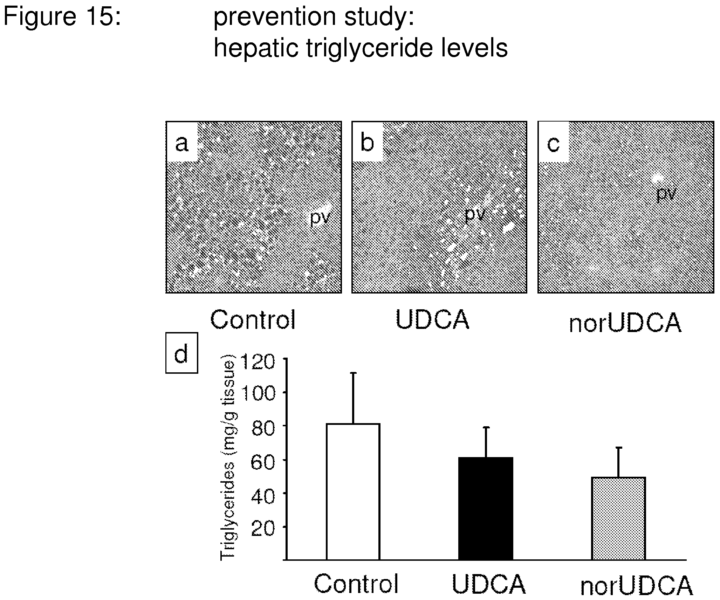

Figure 14: experimental setup for data of prevention studies described in Experiment 4 and as depicted in figures 15 to 20
   A: experimental setup; B: body weights of mice of the three different groups over the weeks of treatment
Figure 15: Nor-UDCA reduces hepatic triglyceride levels in a prevention study
   Top: Histological preparations of the liver from mice of groups as indicated wherein neutral fatty acids were stained with oil-red; Bottom: the hepatic triglyceride levels were determined for 5 mice of each group as outlined in figure 14.

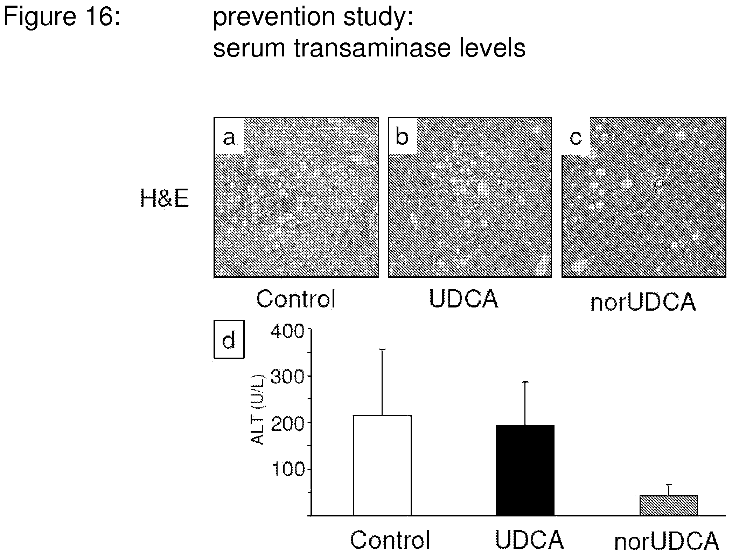

Figure 16: Nor-UDCA reduces ALT-levels in a prevention study
   Top: Histological preparations of the liver from mice of groups as indicated wherein cell nuclei and cytosol were stained with H&E; Bottom: the serum alanin-amino-transferase levels were determined for 5 mice of the group fed with western diet only, for 5 mice of the group fed with western diet comprising additionally nor-UDCA and for 5 mice of the group fed with western diet comprising additionally UDCA.

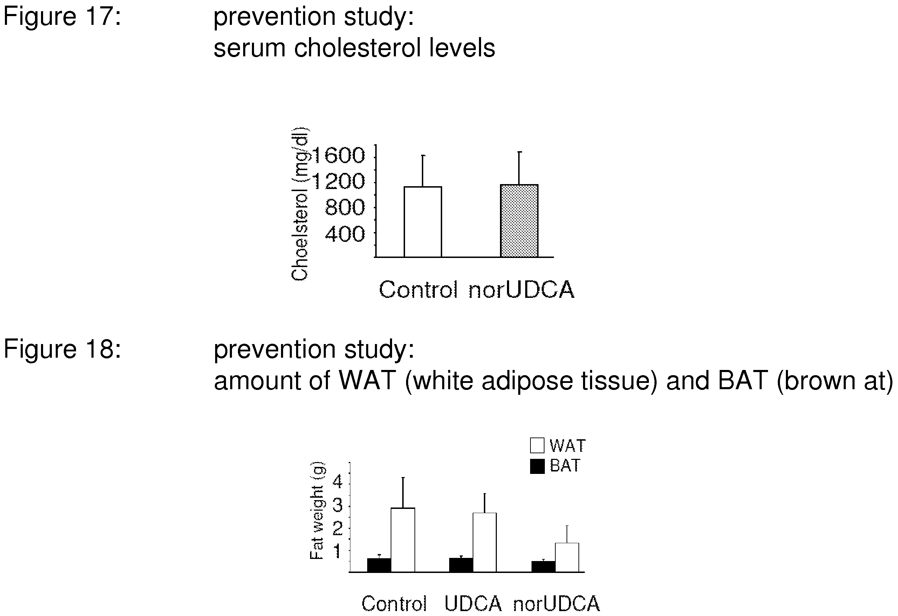

Figure 17: Nor-UDCA does not seem to reduce serum cholesterol levels in a prevention study The serum cholesterol levels were determined for 5 mice of the group fed with western diet only and for 5 mice of the group fed with western diet comprising additionally nor-UDCA.
Figure 18: Nor-UDCA reduces the amount of white adipose tissue in a prevention study The weights of either the brown adipose tissue (BAT) or the white adipose tissue (WAT) were determined for 5 mice of each group and are depicted as fat weight in gram.

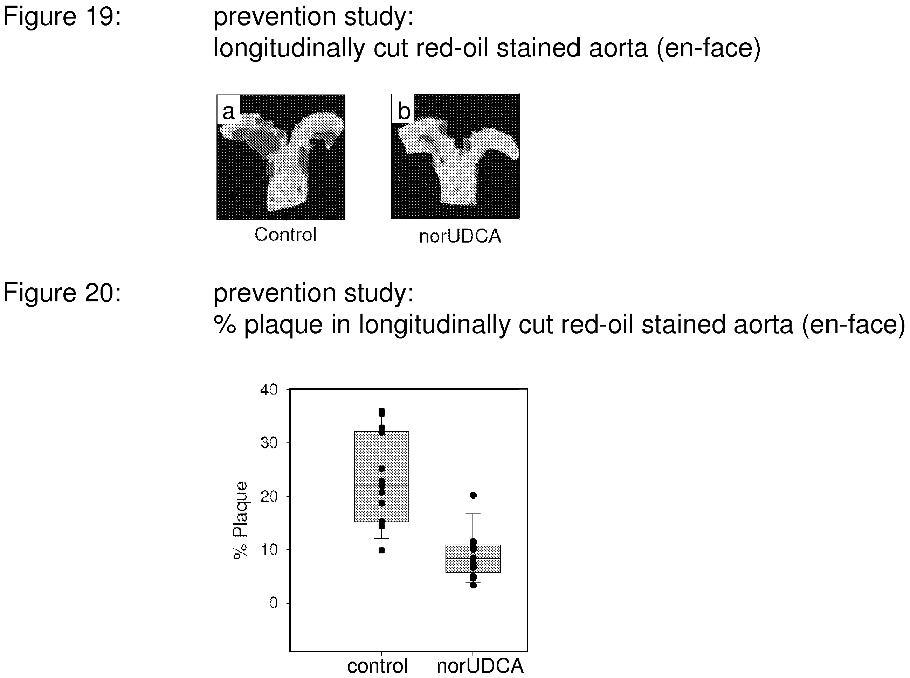

Figure 19: Nor-UDCA reduces UDCA reduces plaques in the aorta in a prevention study Longitudinal sections of the aorta were en-face stained with red oil for the control group (a) and for the group fed additionally with nor-UDCA for prevention (b). Furthermore, the area comprising plaques was determined and expressed as % plaques of the total area. The analysis was done for 10 mice in each group as depicted.

### DETAILED DESCRIPTION OF THE INVENTION

The present invention lies in the surprising finding that one can use nor-ursodeoxycholic acidand/or its pharmaceutically acceptable salts and esters for the treatment and/or prevention of arteriosclerosis.

In the context of the present disclosure, it needs to be understood that the treatment and/or prevention of arteriosclerosis may comprise the treatment and/or prevention of risk factors for arteriosclerosis and particularly atherosclerosis as well. In this regard, the treatment and/or prevention of NAFLD as one risk factor is preferred.

Before some of the embodiments of the present invention are described in further detail, the following definitions are introduced.

As used in the specification and in the claims, the singular forms of "a" and "an" also include the respective plurals unless the context clearly dictates otherwise.

The terms "about" and "approximately" in the context of the present invention generally denote a level or interval of accuracy that a person skilled in the art will understand to still ensure the technical effect of the feature in question. As regards numerical values, these terms typically indicate a deviation from the indicated numerical value of ±10% and preferably of ±5%.

It is to be understood that the term "comprising" is not limiting. For the purposes of the present invention, the term "consisting of' is considered to be a preferred embodiment of the term "comprising of'. If hereinafter a group is defined to comprise at least a certain number of embodiments, this is also a means to disclose a group, which preferably consists of these embodiments only.

Similarly, if in the context of the present invention, a group is recited to comprise "at least one" embodiment, it also means to disclose a group, which preferably consists of the one embodiment only that is specifically mentioned.

Further definitions of terms will be given in the context of which these terms are used.

As mentioned above, the present invention is based on the finding that one can use at least nor-ursodeoxycholic acidand/or at least one pharmaceutically acceptable salt and/or ester thereof for the treatment and/or prevention of arteriosclerosis.

The term "pharmaceutical acceptable salt" as used herein, includes acid addition salts as well as base addition salts.

Suitable pharmaceutically acceptable acid addition salts may include salts made from inorganic acids such as the chloride, bromide, iodide, sulfate, bisulfate, phosphate, acid phosphate, nitrate salt. Acid addition salts may also include salts from organic acids such as the citrate, oxalate, isonicotinate, lactate, salicylate, tartrate, oleate, fumarate, tannate, pantothenate, bitartrate, ascorbate, succinate, maleate, gentisinate, fumarate, gluconate, glucoronate, saccharate, formiate, benzoate, glutamate, aspartate, methanesulfonate, ethanesulfonate, benzensulfonate, p-toluensulfonate and parmoate salt.

Other organic acids including aliphatic, cycloaliphatic, aromatic, heterocyclic, carboxylic and sulfonic classes of organic acids from which pharmaceutically acceptable salts of nor-bile acids can be made include propionic, glycolic, pyruvic, anthranilic, mandelic, mesylic, p-hydroxy benzoic, phenylacetic, 2-hydroxyethane sulfonic, alginic, sulfanilic, stearic, p-hydroxybutyric, cyclohexylaminosulfonic, galactaric and galacturonic acid and the like.

Suitable bases for formation of base addition salts include, but are not limited to hydroxides of alkali metals such as sodium, potassium and lithium; hydroxides of alkaline earth metals such as calcium and magnesium; hydroxides of other metals such as aluminium and zinc, ammonia and organic amines such as unsubstituted or hydroxyl-substituted mono-, di-, or trialkyl amines; dicyclohexylamine; tributylamine; pyridine; N-methyl, N-ethylamine; diethylamine, triethylamine; mono-, bis-, or tris-(2-hydroxy lower alkyl amines) such as mono-, bis- or tris-(2-hydroxy ethyl) amine, 2-hydroxy-tert-butylamine or tris-(hydroxymethyl) methyl amine; N, N-di-lower alkyl-N(hydroxyl lower alkyl)-amines such as N, N-dimethyl-N(2-hydroxyethyl)amine or tri-(2-hydroxyethyl)amine and N-methyl-D-glucamine. Other bases, which can be used to form base addition salts, are e.g. amino acids such as arginine, lysine and the like.

In the context of the present invention the term "pharmaceutically acceptable esters" are nontoxic esters of nor-bile acids as mentioned above and preferably alkyl esters such as methyl, ethyl, propyl, isopropyl, butyl, isobutyl or pentyl esters as well as aryl esters. Esterification of carboxylic acids such as nor-UDCA can be performed by procedures, as they are commonly known in the art. Typical esters of nor-UDCA comprise e.g. a acetatemethyl ester of nor-UDCA (PubChem Substance ID (SID) 10543236) or a trimethylsilylethermethylester of nor-UDCA (PubChem SID 10492328). The public PubChem database can be found at http://pubchem.ncbi.nlm.nih.gov/.

The term "pharmaceutically acceptable derivative" refers to the taurine and glycine esters of nor-bile acids as nor-UDCA. Other derivatives of nor-bile acids include sulphate or glucoronide forms of nor-bile acids such as nor-UDCA.

In the context of the present invention, the above-mentioned pharmaceutically acceptable salts, esters and/or derivatives refer to nor-UDCA. Nor-UDCA may preferably be administered as the free acid.

Nor-UDCA and/or a pharmaceutically acceptable salt and/or ester thereof may be used in an amount of 10 to 8,000 mg, 25 to 5,000 mg, 50 to 1,500 mg or 250 to 500 mg.

As regards human patients, nor-UDCA and/or pharmaceutically acceptable salts and/or esters thereof may be administered to a patient in an amount of about 25 mg to 5,000 mg, preferably of about 100 mg to about 2,500 mg per day. A human patient may in particular be treated with about 800 mg to about 1,500 mg and more specifically with about 1,000 mg per day of nor-UDCA and/or pharmaceutically acceptable salts and/or esters thereof.

Another suitable criterion for selecting an appropriate amount of nor-UDCA and/or of a pharmaceutically acceptable salt and/or ester thereof is that nor-UDCA and/or pharmaceutically acceptable salts and/or esters thereof may be administered to an individual in an amount of about 1 to about 100 mg/kg/d, preferably in an amount of about 5 to about 50 mg/kg/d, more preferably in an amount of about 10 to about 25 mg/kg/d and in particular in an amount of about 12 to about 15 mg/kg/d.

These amounts can be administered at once or as multiple doses (at least 2, 3, 4, 5 or 10 doses) per day.

The term "arteriosclerosis" is used in the context of the present invention by its regular meaning, i.e. conditions that involve the hardening of arteries.

A specific condition falling under the common definition "arteriosclerosis" is the treatment and/or prevention of arteriolosclerosis, which refers to conditions in which the small arteries are affected. The term "arteriolosclerosis" is also used as it is commonly applied in the art.

The invention relates to the application of nor-UDCA and/or pharmaceutically acceptable salts and esters thereof in the treatment of atherosclerosis.

The term "atherosclerosis" is used in the context of the present invention to have the same meaning as it is commonly used in the art. Thus, the term "atherosclerosis" describes the whole series of steps including the deposition of lipids, white blood cells such as leucocytes, monocytes and macrophages in arteries leading ultimately to atheotmatous plaque formations in the vessel walls of arteries.

The terms "treatment of arteriosclerosis" and preferably "atherosclerosis" indicate that existing arteriosclerosis and/or atherosclerosis can be improved by application of nor-UDCA and/or pharmaceutically acceptable salts, esters or derivatives thereof. Improvement in the context of the present invention can mean that e.g. atheromatous plaque size and/or frequency is reduced. Improvements also can relate to a reduction of hepatic neutrophil granulocytes, a reduction of hepatic triglyceride levels, a reduction of serum cholesterol levels and a reduction of white adipose tissue.

The terms "treatment of arteriosclerosis" and preferably "atherosclerosis" also refer to an improvement of key manisfestations thereof such as coronary artery disease, cerbrovascular disease and peripheral vascular disease.

The terms "prevention of arteriosclerosis" and preferably "of atherosclerosis" mean that administration of nor-UDCA and/or pharmaceutically acceptable salts and esters thereof reduce the likelihood of development of these conditions or at least alleviate the extent and/or frequency to which these diseases develop.

The terms "prevention of arteriosclerosis" and preferably "of atherosclerosis" also mean that administration of nor-UDCA and/or pharmaceutically acceptable salts and esters thereof reduce the likelihood of development of key manisfestations thereof such as coronary artery disease, cerbrovascular disease and peripheral vascular disease.

As set out above, the disclosure also relates to the treatment and/or prevention of risk factors for arteriosclerosis. In one aspect, the disclosure relates to the treatment and/or prevention of NAFLD as one risk factor.

In another aspect, the present disclosure relates to the treatment and/or prevention of arteriosclerosis and particularly atherosclerosis in combination with the treatment and/or prevention of NAFLD by one single class of compounds, namely nor-bile acids with nor-UDCA being preferred. Thus, NAFLD and atherosclerosis may be treated by one single class of compounds, preferably by nor-UDCA.

The term "NAFLD" is used in the context of the present disclosure by its regular meaning, i.e. the non-alcoholic hepatic steatosis with a broad spectrum of clinical and pathological manifestations and conditions such as abdominal obesity, type 2 diabetes, insulin resistance, hypertension and dyslipidaemia.

The term "treatment of NAFLD" indicates that parameters of NAFLD such as high fatty acid concentrations in the liver (e.g. triglycerides) or high serum transaminase levels (e.g. alanin-amino-transferase) improve by application of nor-bile acids and/or pharmaceutically acceptable salts, esters and derivatives thereof and preferably of nor-UDCA and/or pharmaceutically acceptable salts, esters and derivatives thereof.

The term "prevention of NAFLD" means that the likelihood of development of typical manifestations of NAFLD as set out above is reduced by the administration of nor-bile acids and/or pharmaceutically acceptable salts, esters and derivatives thereof and preferably of nor-UDCA and/or pharmaceutically acceptable salts, esters and derivatives thereof.

In order to determine whether an improvement in e.g. atherosclerosis or a preventive effect in atherosclerosis is achieved by application of nor-UDCA and/or pharmaceutically acceptable salts and esters thereof, one will make a comparison with a control which may e.g. be a human subject for which diagnosis indicates that it is not suffering from e.g. atherosclerosis. In a preferred embodiment the improved effects during treatment and/or the improved preventive effect will be determined with respect to a control group that is under medical treatment with one of the medications that are currently used for e.g. treatment of atherosclerosis such as statins.

In order to determine e.g. whether plaque-formation in atherosclerosis has been affected by application of nor-UDCA and/or pharmaceutically acceptable salts and esters thereof, one may rely on the common diagnostic methods known in the art, such as e.g. histopathological examinations.

The medicaments or pharmaceutical dosage forms which comprise at least nor-UDCA and or at least one pharmaceutically acceptable salt and ester thereof are formulated for oral application.

Pharmaceutical dosage forms may be solid or liquid dosage forms or may have an intermediate, e.g. gel-like character depending on the route of administration and other objectives.

According to the invention, oral dosage forms are used, wherein the oral dosage form is a controlled release dosage form that releases at least nor-UDCA and /or at least one pharmaceutically acceptable salt and/or ester thereof only after the oral dosage form has reached the stomach or the gastro-intestinal tract. Oral dosage forms are used in view of patients' overall acceptance of this type of dosage forms.

Oral dosage forms may be liquid or solid.

Solid oral dosage forms can include e.g. tablets, troches, pills, capsules, powders and granules.

In one embodiment, the oral dosage forms may be formulated to ensure a controlled release of nor-UDCA and/or its pharmaceutically acceptable salts, esters and/or derivatives. Such dosage forms may therefore be designated as controlled release (CR) pharmaceutical dosage forms.

The term "controlled release dosage form" in the context of the present invention is used to highlight that a pharmaceutical dosage form is not an immediate release (IR) pharmaceutical dosage form. An oral immediate release pharmaceutical dosage form will typically release substantially all of the at least one nor-bile acid and/or its pharmaceutically acceptable salts, esters and/or derivative thereof within a short time after administration. Typically, an IR dosage form will have released 70% by weight of the pharmaceutically active agents within thirty minutes of administration. The release rates may be determined using the European Pharmacopoeia Paddle Method.

A controlled release dosage form may designate a pharmaceutical dosage form that releases the active agent only after the dosage form has reached a certain site of the body, i.e. the stomach or the gastro-intestinal tract. Additionally or alternatively it may designate a dosage form, which releases the active agent over a prolonged period of time. In the latter case, a controlled release dosage form may be designated as a sustained release dosage form.

A site-specific controlled release of the pharmaceutically active agent, being in the present case nor-UDCA or pharmaceutically acceptable salts, esters and/or derivatives thereof, may e.g. achieved in that the release is made dependent on the pH value of the liquids that the dosage form encounters when passing through the human body. Such a pH-dependent release may allow that a dosage form releases the active agent not in the stomach, but only in the gastro-intestinal tract. Another aspect would be that such a controlled release dosage form releases the active agent once it enters the body. A typical example of controlled release dosage form which pH-independently release the active agent are dosage forms that comprise an enteric coating.

The term "sustained release" instead refers to the release of the pharmaceutically active compounds from the dosage form over an extended period of time but not necessarily to the release at a defined place. In general, sustained release in the context of the present invention means that a pharmaceutically active agent such as nor-UDCA and its pharmaceutically acceptable salts, esters and/or derivatives are released from the pharmaceutical dosage form over a time period of at least 2 hours. Of course, the release of the pharmaceutically active agent from the dosage form may take place over time periods of at least 4 hours, at least 6 hours, at least 10 hours, at least 12 hours or at least 14 hours.

The sustained release characteristics of a dosage form may be adapted such that a therapeutic effect for at least 8 hours, for at least 12 hours or for at least 24 hours is achieved. Such pharmaceutical dosage forms have the advantage that they can be administered on a 3-times, 2-times or once-a-day basis to the patient.

Of course, the above principles can be combined. For example, a pharmaceutical dosage form may comprise an enteric coating in order to ensure that the active agent is released only in the gastro-intestinal tract. The release during the gastro-intestinal passage may, however, display the characteristics of sustained release.

According to the disclosure, additionally and/or alternatively the principles of immediate release and sustained release may be combined. Thus, according to the disclosure, a dosage form may comprise an immediate release phase that ensures a quick onset of therapeutic action that is then prolonged by a second phase of the pharmaceutical dosage form ensuring sustained release characteristics.

Sustained release characteristics can be achieved by different formulation approaches. For example, a pharmaceutical dosage form may comprise a sustained release matrix in which the pharmaceutically active agent such as nor-UDCA is embedded in order to achieve the sustained release properties of the dosage form.

In another embodiment, a sustained release coating may be used to ensure the sustained release characteristics of the dosage form. In such a case, the pharmaceutically active agent such as nor-UDCA may be applied on/or within e.g. a carrier, which has no substantial influence on the release of the active agent. This drug-loaded carrier may then be overcoated with a corresponding sustained release coating.

These approaches for achieving sustained release of a pharmaceutically active agent, i.e. use of a matrix or a coating may of course, also be combined. The person skilled in the art is further aware of other technical approaches for achieving a sustained release of the dosage form which include e.g. osmotically driven sustained dosage forms.

Typically, if a sustained release matrix system is used, the pharmaceutically active agent such as nor-UDCA will be dispersed throughout a matrix-forming material. The matrix-forming materials may be chosen to achieve an erosive matrix, a diffusion matrix or a matrix system, which combines the characteristics of an erosive and a diffusion matrix. Suitable materials for inclusion in a sustained release matrix include hydrophilic or hydrophobic polymers including cellulose ethers and preferably alkyl celluloses and hydroxyl alkyl celluloses as well as acrylic resins. Other materials that may be used in a sustained release matrix may be fatty alcohols, fatty acids or polyethylene glycols. The person skilled in the art will be aware of how to build such pharmaceutical dosage forms.

In general, solid dosage forms will comprise various pharmaceutical acceptable excipients which will be selected depending on which functionality is to be achieved for the dosage form. Typical pharmaceutically acceptable excipients include substances like sucrose, manitol, sorbitol, starch and starch derivatives, lactose, and lubricating agents such as magnesium stearate, disintegrants and buffering agents.

In case that liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents.

Further conventional excipients, which can be used in the aforementioned dosage forms depending on the functionality that is to be achieved for the dosage form, include pharmaceutically acceptable organic or inorganic carrier substances which do not react with the active compound. Suitable pharmaceutically acceptable carriers include, for instance, water, salt solutions, alcohol, oils, preferably vegetable oils, polyethylene glycols, gelatin, lactose, amylose, magnesium stearate, surfactants, perfume oil, fatty acid monoglycerides and diglycerides, petroethral fatty acid esters, hydroxymethyl-cellulose, polyvinylpyrrolidone and the like. The pharmaceutical preparations can be sterilized and if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compounds . For parenteral application, particularly suitable vehicles consist of solutions, preferably oily or aqueous solutions, as well as suspensions, emulsions, or implants.

The person skilled in the art is aware that bioavailability of nor-UDCA, pharmaceutically acceptable salts and/or esters thereof can be enhanced by micronisation of the formulations and the actives using conventional techniques such as grinding, milling and spray-drying in the presence of suitable excipients or agents such as phospholipids or surfactants.

The pharmaceutical compositions in accordance with the present invention may not only comprise nor-UDCA and/or pharmaceutically acceptable salts and/or esters thereof but also pharmaceutically active agents which are known to have a positive effect on the treatment and/or prevention of arteriosclerosis and preferably of atherosclerosis. These additional pharmaceutically active agents include e.g. statins as well as glitazones and acetic acetylic acid.

Furthermore, as the pharmaceutical compositions in accordance with the disclosure with nor-UDCA being a preferred representative thereof and/or pharmaceutically acceptable salts and/or esters thereof have a positive effect on NAFLD, they may also be used in combination with pharmaceutically active agents which are on the one hand known to have a positive effect on the treatment and/or prevention of arteriosclerosis and preferably of atherosclerosis but are on the other hand also known to induce steatosis of the liver, such as e.g. LXR antagonists or the like.

However, as set out above, the pharmaceutical compositions in accordance with the disclosure with nor-UDCA being a preferred representative thereof and/or pharmaceutically acceptable salts and/or esters thereof may also be used as single active agent having a positive effect on the treatment and/or prevention of arteriosclerosis and preferably of atherosclerosis on the one hand, and on the treatment and/or prevention of NAFLD on the other hand.

A pharmaceutical composition comprising at least nor-UDCA and/or pharmaceutically acceptable salts, esters and/or derivatives thereof may be especially used for the treatment and/or prevention of atherosclerosis by at least partially inhibiting and/or partially actively reversing a plaque formation.

Further preferred embodiments of the disclosure relate to:
1. Use of at least nor-ursodeoxycholic acid and/or at least one pharmaceutically acceptable salt and/or ester thereof in the manufacture of a medicament for the treatment and/or prevention of arteriosclerosis.
2. Use according to 1 wherein the medicament is used for treatment and/or prevention of arteriolosclerosis and/or preferably of atherosclerosis.
3. Use according to 1 or 2 wherein at least nor-ursodeoxycholic acid or at least one pharmaceutically acceptable salts and/or esters thereof is used.
4. Use according to 3 wherein the medicament comprises a pharmaceutically effective amount of at least nor-ursodeoxycholic acid or at least one pharmaceutically acceptable salt, ester and/or derivative thereof and optionally at least one pharmaceutically acceptable excipient.
5. Use according to 4 wherein the medicament comprises about 10 to about 8,000 mg, preferably about 25 to about 5,000 mg, more preferably about 50 to about 1,500 mg and most preferably about 250 to about 500 mg of at least nor-ursodeoxycholic acid or of at least one pharmaceutically acceptable salt, ester and/or derivative thereof.

The present invention will now be illustrated with respect to examples that are, however, not to be construed as being limiting.

### Experiment 1 - treatment of ApoE-/- mice with nor-UDCA

In order to study the effects of nor-UDCA on atherosclerosis, ApoE-deficient mice were treated. To this end ApoE-/- mice as described in Piedrahita et. al. (PNAS, 1992, 89, 4471-4475) which carry the *Apoe^{tm1Unc}* mutation were back-crossed 10 times into the C57BL76J. ApoE-deficient mice can also be obtained from the Jackson Laboratory (Maine, US) with the strain name *B6.129P2.-Apoe^{tm1Unc}*/*J*(http://jaxmice.jax.org).

ApoE-/- mice (n = 10) were fed with normal diet for 8 weeks post natum, followed by western chow diet for another 8 weeks. The western chow diet consists of 21 % by weight fat, 1.5 % by weight cholesterol and no cholic acid ("high fat diet").

This type of western chow diet is known to force atherosclerotic plaque formation on the indicated time line in ApoE-/- mice. After eight weeks of being fed with western chow diet, the mice were segregated into two groups with each group comprising five mice. In the control group A, the mice were further fed with the western chow diet for additional four weeks. In the other group B, the ApoE-/- mice were fed with a western chow diet comprising 0.5% w/w nor-UDCA for additional four weeks. Considering a food intake of 4g/day of chow per mouse, this corresponds to 20 mg/day of nor-UDCA per mouse.

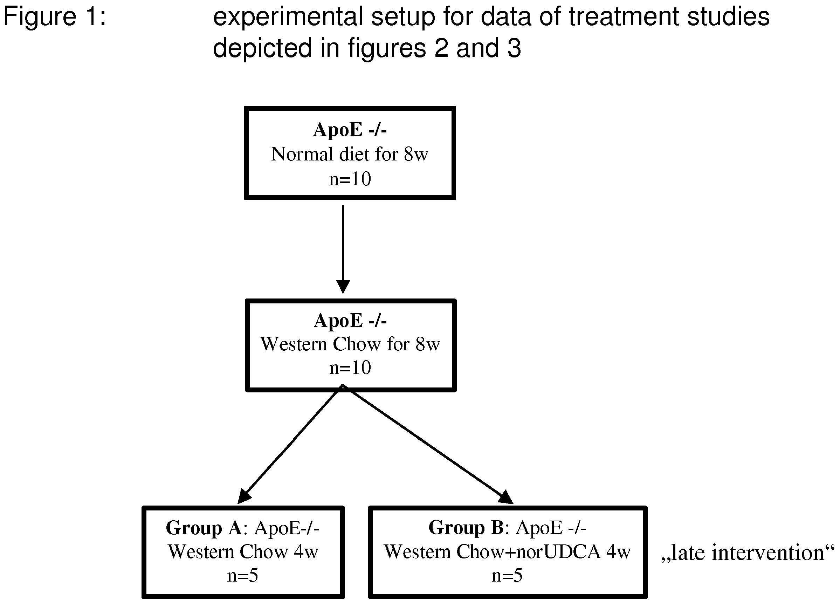

The experimental setup is depicted in Figure 1.

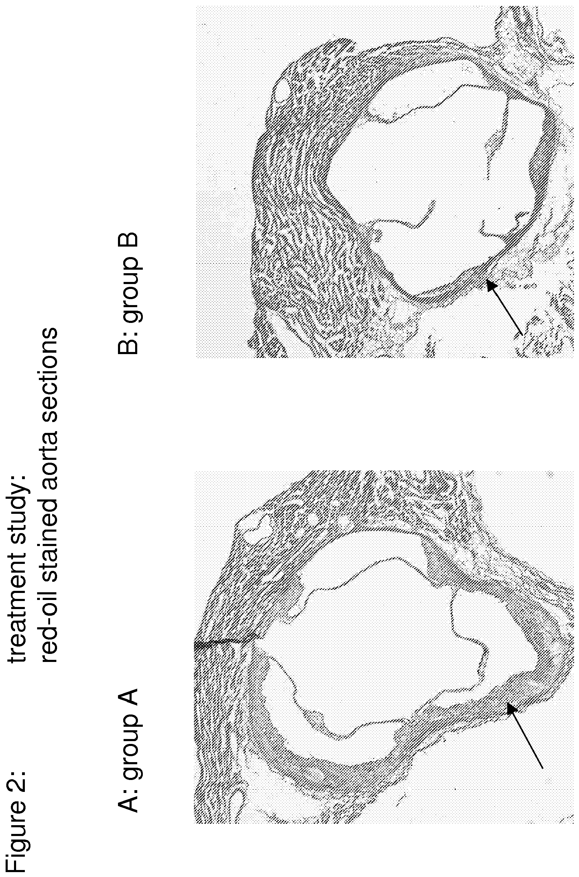

Subsequently these mice were analysed 20 weeks post natum by different means as shown in Figures 2 and 3.

Figure 2 shows a histological analysis. Depicted are cross-sectional views of red-oil stained aorta sections in ApoE-/- mice. One can clearly see the development of arteriosclerotic plaques in control mice (A) while the nor-UDCA-fed mice show a significantly reduced extent of these plaques (B).

Figure 3 then represents a morphometric analysis in which the sclerotic area versus the aorta area is determined. One can clearly see that the sclerotic area is significantly smaller in the nor-UDCA ApoE-/-mice (group B). The number of mice analysed was 5 for each group.

### Experiment 2 - treatment of LDLR-/- mice with nor-UDCA

In a second experiment (Fig. 4 and 5), the effect of nor-UDCA in LDLR-/- mice that were raised on a western chow diet was investigated.

As for the ApoE-deficient mice, the LDLR-deficient mice are considered to be a bona-fide animal model of atherosclerosis development. If these mice are raised on a western chow diet they evolve typical symptoms of atherosclerosis involving atherosclerotic plaque formation in the aorta (Fig.5).

LDLR-/- mice were produced as described in Ishebashi et. al. (J.Clin. Jnnvest., 1993, 92, 883-893). The 129-derived AB1 ES cell line was used. The strain was backcrossed to C57BL/6J mice for 10 generations. Ld1r^{tm1Her}/Ld1r^{tm1Her} involves: 129S7/SvEvBrd * C57BL/6. LDLR-deficient mice can also be obtained from the Jackson Laboratory (Maine, US) with the strain name B6.129S7-*Ldblr^{tm1Her}*/J (http://jaxmice.jax.org).

As in Experiment 1, ten mice being deficient in LDLR were raised on normal diet during the first 8 weeks post natum followed by the western chow diet for another eight weeks.

Afterwards, the ten mice were separated into two groups for which the control group A received further feeding on a western chow diet for 4 weeks. In the test group B, the western chow diet of the following 4 weeks was supplemented with 0.5% w/w nor-UDCA. Considering a food intake of 4g/day of chow per mouse, this corresponds to 20mg/day of nor-UDCA per mouse.

The study design is depicted in Figure 4.

Subsequently these mice were analysed 20 weeks post natum as shown in Figure 5:
A histological analysis of the aorta of LDLR-/- mice was performed. To this end a longitudinal section of the aorta was en-face stained with red oil. From Figure 5, one can clearly see that the extent and size of atherosclerotic plaque is significantly reduced in the mice of group B.

One can clearly see from experiments 1 and 2 that nor-UDCA reduces the size of atherosclerotic plaques in animal models, which are considered to be *bona fide* representatives for atherosclerotic development.

### Experiment 3 - treatment of ApoE-/- mice with nor-UDCA in comparison to UDCA

ApoE-deficient mice as described in Experiment 1 were used.

ApoE-/- mice (n = 15) were fed with normal diet for 8 weeks post natum, followed by western chow diet ("high fat diet") for another 8 weeks. The mice were then segregated into three groups with each group comprising five mice. In the control group, the mice were further fed with the western chow diet for additional four weeks. In the second group, the ApoE-/- mice were fed with a western chow diet comprising 0.5% w/w nor-UDCA for additional four weeks. Finally, in the third group, the ApoE-/- mice were fed with a western chow diet comprising 0.5% w/w UDCA for additional four weeks. Considering a food intake of 4g/day of chow per mouse, this corresponds to 20 mg/day of nor-UDCA or UDCA, respectively, per mouse.

The experimental setup is depicted in Figure 6.

Subsequently these mice were analyzed 20 weeks post natum by different means as shown in Figures 7 to 13.

Figure 7 shows the effect of either UDCA or nor-UDCA on the number of hepatic neutropohil granulocytes. Said cells are indicative for an inflammation in the liver and, thus, for the presence of inflammatory signals in general. In the top of Figure 7, an immunohistochemical analysis of liver tissue of the three different groups a-c is shown. In each case, preparations of liver tissue were first incubated with an antibody specific for surface-antigen CD11b, which is present on the surface of neutropohil granulocytes only. Following said incubation, the anti-CD11b antibody was detected with a secondary antibody-conjugate resulting in the stain of CD11b-positive cells (see arrows ). Clearly, the number of hepatic neutropohil granulocytes is reduced in mice fed with nor-UDCA (c). Below the stains, a quantitative analysis is shown. To this aim, CD11b-stained liver preparations of 5 mice of each group were analyzed: In each preparation, CD11b positive cells were counted in 30 different areas of the preparation using a microscope with a magnification of x20. The cell number was then averaged for one single area in each preparation and subsequently for one single area of the 5 preparations / group. Again, the number of hepatic neutropohil granulocytes is clearly reduced in mice fed with nor-UDCA. Thus, nor-UDCA exhibits an anti-inflammatory effect.

Figure 8 shows an analysis of the expression level of p-JNK in liver tissue. The expression level of the p-JNK-kinase is indicative for the presence of inflammatory signals as p-JNK is part of the inflammatory signaling in cells. To this aim, an identical amount of liver tissue of three mice (corresponding to different lanes in the WB) of either normally fed ApoE-deficient mice (ApoE) or of the three groups as outlined above (ApoE + WD, ApoE + WD + UDCA, ApoE + WD + nor-UDCA) was homogenized and cell lysates were prepared according to standard methods. Said lysates were normalized for the amount of protein present according to standard methods. Fractions containing identical amounts of protein were then analyzed by standard Western-blot techniques (denaturation of the samples by adding SDS-buffer and boiling followed by SDS-PAGE and transfer to a membrane; quantitative analysis of the proteins bound to the membrane by incubation with a first (anti-p-JNK 1/2) and second (directed against the first antibody, used as conjugate) antibody followed by chemo luminescent detection of the second antibody-conjugate) for the amount of p-JNK. Clearly, nor-UDCA is capable of downregulating the expression of p-JNK (to "normal" levels, see ApoE-control in the left lanes!) in contrast to UDCA.

Figure 9 shows the hepatic triglyceride levels of the three different populations. For each population, 5 mice were analyzed and the depicted values represent the averaged triglyceride amounts. An identical amount of liver tissue of each mouse was homogenized and the amount of triglycerides present in each homogenate was determined by standard methods. Clearly, nor-UDCA is capable of significantly reducing the triglyceride-amount in the liver when compared to the control or to the UDCA-treated mice.

Figure 10 shows the serum transaminase levels (in this case alanin-amino-transferase) as indicators for the condition of liver cells (the more liver cells die, the higher the serum transaminase levels). To this aim, the activity of the alanin-amino-transferase present in a defined volume of a blood sample was determined by standard diagnosis-laboratory methods and given as enzymatic activity of Units / liter. 5 mice were used either in the control or the nor-UDCA-treated population and the depicted values represent the averaged ALT-activities.

Nor-UDCA shows a trend of reducing the serum transaminase levels compared to the control and, thus, seems to have a positive impact on liver cells.

Figure 11 shows the total serum cholesterol levels. To this aim, the amount of serum cholesterol in a defined volume of a blood sample was determined by standard diagnosis-laboratory methods and is shown in mg/dl. 5 mice were used either in the control or the nor-UDCA-treated population and the depicted values represent the averaged cholesterol amounts. Nor-UDCA seems to be capable of lowering the total cholesterol compared to the control. The positive effects of nor-UDCA on atherosclerosis, however, seem to be mainly independent of the overall serum cholesterol levels.

Figure 12 represents a quantitative analysis wherein the area comprising plaques versus the total area in the aortic arch is determined. To this aim, a histological analysis was performed followed by a quantification. Firstly, a longitudinal section of the aorta was en-face stained with red oil (in each group for 5 mice). In a second step, the area comprising plaques was determined and, using the total area, said plaque-area was expressed in % plaques in the aortic arch (the depicted values represent the averaged counts or percent, respectively, for 5 mice in each group). Nor-UDCA seems to be as effective as UDCA with both substances decreasing the percent of plaques.

Figure 13 shows a histological analysis (top). Depicted are cross-sectional views of red-oil stained aortic valves prepared from mice as indicated. Clearly, the amount of plaques is reduced in both the UDCA and the nor-UDCA treated mice. Consistent with this result, also a quantification of the plaque area versus the total surface area (expressed as plaques in % of the surface area) shows a significant reduction of said % plaques in nor-UDCA-treated mice. 5 samples of each population were used for said quantification.

Summarizing the data gained in the treatments studies as outlined above, nor-UDCA results in
1. a reduction of the number, the extent and the size of atherosclerotic plaques, most likely via a direct effect on the plaques (and macrophages, respectively) and not via a reduction of serum cholesterol levels
1. a reduction of hepatic triglyceride and serum transaminase levels
2. a reduction of inflammatory signals and of cells induced by such inflammatory signals in the liver.

Thus, nor-UDCA reduces atherosclerotic plaques, has positive effects on the non-alcoholic fatty liver during treatment of atherosclerosis and acts anti-inflammatory.

### Experiment 4 -prevention of atherosclerosis in ApoE-/- mice by nor-UDCA in comparison to UDCA

ApoE-deficient mice as described in Experiment 1 were used.

ApoE-/- mice (n = 15) were fed with normal diet for 8 weeks post natum and then segregated into three groups with each group comprising five mice. In the control group, the mice were fed with the western chow diet for additional 12 weeks. In the second group, the ApoE-/- mice were fed with a western chow diet comprising 0.5% w/w nor-UDCA for additional 12 weeks. Finally, in the third group, the ApoE-/- mice were fed with a western chow diet comprising 0.5% w/w UDCA for additional 12 weeks. Considering a food intake of 4g/day of chow per mouse, this corresponds to 20 mg/day of nor-UDCA or UDCA, respectively, per mouse.

Thus, in this experimental prevention setup, the mice are fed with diet known to force atherosclerotic plaque formation together with either UDCA or nor-UDCA (mice of the second and third group) having preventive effects on the plaque formation. When compared to a control fed with western chow diet only, the preventive effects can be studied.

The experimental setup is depicted in Figure 14 (a).

Figure 14(b) depicts the body weights of the animals of the three groups (5 per group with groups as indicated in (a)) over a period of the treatment as indicated. Neither of the therapies as depicted in a) had a significant influence on the body weights of the mice as the body weights do not significantly vary among the groups. Thus, all effects shown below cannot be attributed to the body weights of the animals.

Subsequently these mice were analyzed 20 weeks post natum by different means as shown in Figures 15 to 20.

Figure 15 shows the hepatic triglyceride levels for the three different populations. Figures a)-c) are histological preparations of the liver wherein neutral fatty acids (mainly triglycerides) were stained with oil-red. Clearly, the hepatic triglyceride amounts are decreased in preparation c) of a mouse fed with additional nor-UDCA. In the quantification d) below the preparations, 10 mice of each group were analyzed and the depicted values represent the averaged triglyceride amounts. An identical amount of liver tissue of each mouse was homogenized and the amount of triglycerides present in each homogenate was determined by standard methods. Clearly, nor-UDCA is capable of significantly reducing the triglyceride-amount in the liver when compared to the control or to the UDCA-treated mice.

Figure 16 shows a stain of liver cells in general (a) - c)) and the serum transaminase levels (in this case alanin-amino-transferase) as indicators for the condition of liver cells (the more liver cells die, the higher the serum transaminase levels) in d). Figures a)- c) are histological preparations of the liver wherein cells are stained with Heamatoxilin & Eosin stain.

Furthermore, the activity of the alanin-amino-transferase present in a defined volume of a blood sample was determined by standard diagnosis-laboratory methods and given as enzymatic activity of Units / liter (d). 5 mice were used either in the control, the UDCA or the nor-UDCA-treated population and the depicted values represent the averaged ALT-activities. Nor-UDCA significantly reduces the serum transaminase levels compared to the control and UDCA and, thus, seems to have a positive impact on liver cells. This result is clearly supported by the general appearance of the liver cells stained with H&E of figure c).

Figure 17 shows the total serum cholesterol levels. To this aim, the amount of serum cholesterol in a defined volume of a blood sample was determined by standard diagnosis-laboratory methods and is shown in mg/dl. 5 mice were used either in the control or the nor-UDCA-treated population and the depicted values represent the averaged cholesterol amounts. The positive effects of nor-UDCA on atherosclerosis seem to be independent of the overall serum cholesterol levels.

Figure 18 shows the influence of nor-UDCA versus control and UDCA on the weight of white and brown adipose tissue of the mice. To this aim, the weight of the corresponding adipose tissue of each mouse was determined. 5 mice were used per group and data gaines was averaged over said population. Clearly, neither UDCA nor nor-UDCA had an influence on the weight of the brown adipose tissue. Nor-UDCA, however, showed a strong reduction of the amount of white adipose tissue compared to the control or the UDCA-treated mice. Thus, nor-UDCA has a positive effect on the reduction of the amount of white adipose tissue.

In Figure 19, a histological analysis of the aorta is shown in the two preparations on the top. For this analysis, a longitudinal section of the aorta was en-face stained with red oil fore lipid plaques. Comparing said two preparations, one can clearly see that the extent and size of atherosclerotic plaques is significantly reduced in the mouse being fed with additional nor-UDCA (b). Figure 20 then represents a morphometric analysis in which the sclerotic area versus the total aorta area is determined in preparations according to Figure 19. In each group, 10 mice were analyzed and each dot on the blot of the control or the "nor-UDCA supplemented" fed mice corresponds to the result for one mouse. One can clearly see that the sclerotic area is significantly smaller in the nor-UDCA fed ApoE-/-mice.

Summarizing the data gained in the prevention studies as outlined above, nor-UDCA results in
1. a reduction of the number, the extent and the size of atherosclerotic plaques, most likely via a direct effect on the plaques (and macrophages, respectively) and not via a reduction of serum cholesterol levels
2. a reduction of hepatic triglyceride and serum transaminase levels as well as in the amount of white adipose tissue

Thus, nor-UDCA reduces atherosclerotic plaques and has positive effects on the non-alcoholic fatty liver during prevention of atherosclerosis.

Summarizing the data gained in the treatment and prevention studies, it seems that nor-UDCA exerts its positive effects by a direct effect on macrophages resulting in an increase of cholesterol-export from the macrophages in the serum and thus in a reduction of plaques (as macrophages are "part of" the plaques in the form of foam cells) and by a positive effect on liver enzymes responsible for metabolizing reverse transported serum cholesterol resulting in a higher metabolism of cholesterol in the liver, thus improving the non-alcoholic fatty liver.

## Claims

1. Oral dosage form comprising at least nor-ursodeoxycholic acid and/or at least one pharmaceutically acceptable salt and/or ester thereof for use in the treatment and/or prevention of arteriosclerosis, wherein the oral dosage form comprises 10 to 8,000 mg or 1 to 100 mg/kg/d of at least nor-ursodeoxycholic acid or of at least one pharmaceutically acceptable salt and/or ester thereof, wherein the oral dosage form is a controlled release dosage form that releases at least nor-ursodeoxycholic acid and/or at least one pharmaceutically acceptable salt and/or ester thereof only after the oral dosage form has reached the stomach or the gastro-intestinal tract.

2. Oral dosage form for use according to claim 1, wherein the oral dosage form further comprises at least one pharmaceutically acceptable excipient.

3. Oral dosage form for use according to any one of claims 1 or 2, wherein the oral dosage form comprises about 25 to about 5,000 mg, preferably about 50 to about 1,500 mg and more preferably about 250 to about 500 mg of at least nor-ursodeoxycholic acid or of at least one pharmaceutically acceptable salt and/or ester thereof.

4. Oral dosage form for use according to any of claims 1 to 3, wherein the oral dosage form is used for treatment and/or prevention of arteriolosclerosis and/or preferably of atherosclerosis.

## Patentansprüche

1. Orale Dosierungsform umfassend mindestens nor-Ursodesoxycholsäure und/oder mindestens ein pharmazeutisch akzeptables Salz und/oder Ester davon zur Verwendung bei der Behandlung und/oder Prävention von Arteriosklerose, wobei die orale Dosierungsform 10 bis 8000 mg oder 1 bis 100 mg/kg/Tag von mindestens nor-Ursodesoxycholsäure oder von mindestens einem pharmazeutisch akzeptablen Salz und/oder einem Ester davon umfasst, wobei die orale Dosierungsform eine kontrolliert-freisetzende Dosierungsform ist, die mindestens nor-Ursodesoxycholsäure und/oder mindestens ein pharmazeutisch akzeptables Salz und/oder Ester davon nur freisetzt nachdem die orale Dosierungsform den Magen oder Gastrointestinaltrakt erreicht hat.

2. Orale Dosierungsform zur Verwendung gemäß Anspruch 1, wobei die orale Dosierungsform zusätzlich mindestens einen pharmazeutisch akzeptablen Hilfsstoff umfasst.

3. Orale Dosierungsform zur Verwendung gemäß Anspruch 1 oder 2, wobei die orale Dosierungsform ungefähr 25 bis ungefähr 5000 mg, bevorzugt ungefähr 50 bis ungefähr 1500 mg und noch bevorzugter ungefähr 250 bis ungefähr 500 mg von mindestens nor-Ursodesoxycholsäure oder von mindestens einem pharmazeutisch akzeptablen Salz und/oder Ester davon umfasst.

4. Orale Dosierungsform zur Verwendung gemäß einem der Ansprüche 1 bis 3, wobei die orale Dosierungsform zur Behandlung und/oder Prävention von Arteriolosklerose und/oder bevorzugt von Atherosklerose dient.

## Revendications

1. Forme posologique orale comprenant au moins de l'acide nor-ursodésoxycholique et/ou au moins un sel pharmaceutiquement acceptable et/ou un de ses esters destinée à être utilisée dans le traitement et/ou la prévention de l'artériosclérose, où la forme posologique orale comprend 10 à 8 000 mg ou 1 à 100 mg/kg/j d'au moins l'acide nor-ursodésoxycholique ou d'au moins un sel pharmaceutiquement acceptable et/ou d'un de ses esters, où la forme posologique orale est une forme posologique à libération contrôlée qui libère au moins de l'acide nor-ursodésoxycholique et/ou au moins un sel pharmaceutiquement acceptable et/ou un de ses esters seulement après que la forme posologique orale a atteint l'estomac ou le tractus gastro-intestinal.

2. Forme posologique orale destinée à être utilisée selon la revendication 1, dans laquelle la forme posologique orale comprend en outre au moins un excipient pharmaceutiquement acceptable.

3. Forme posologique orale selon l'une quelconque des revendications 1 à 2, dans laquelle la forme posologique orale comprend environ 25 à environ 5 000 mg, de préférence environ 50 à environ 1 500 mg et davantage de préférence environ 250 à environ 500 mg d'au moins l'acide nor-ursodésoxycholique ou d'au moins un sel pharmaceutiquement acceptable et/ou d'un de ses esters.

4. Forme posologique orale destinée à être utilisée selon l'une quelconque de revendications 1 à 3, dans laquelle la forme posologique orale est utilisée pour le traitement et/ou la prévention de l'artérioslcérose et/ou de préférence de l'athérosclérose.
